# EUROPEAN PATENT SPECIFICATION

(11) **EP 1 698 320 B1**
(45) Date of publication and mention of the grant of the patent: **09.02.2011**
(21) Application number: 04807687.1
(22) Date of filing: 24.12.2004
(51) Int. Cl.: A61K 8/98, A61K 35/20, A61K 35/74, A61P 17/16, A23C 21/02, A23L 1/30

(54) **Method for moisturizing the skin**
Verfahren zur Befeuchtung der Haut
Méthode pour hydrater la peau

(30) Priority: 25.12.2003 JP 2003429243; 05.11.2004 JP 2004322587
(43) Date of publication of application: 06.09.2006
(73) Proprietor: Calpis Co., Ltd., Shibuya-ku, Tokyo 150-0022 (JP)
(72) Inventor: YOSIMURA, Chiaki Calpis Co., Ltd., Kanagawa 252-0206 (JP); MASUYAMA, Akihiro Calpis Co., Ltd., Tokyo 150-0022 (JP)
(74) Representative: Hallybone, Huw George
(86) International application number: PCT/JP2004/019330
(87) International publication number: WO 2005/063196

(56) References cited:
- WO-A-99/47117
- WO-A1-00/41572
- FR-A- 2 778 565
- JP-A- 10 045 610
- JP-A- 10 095 736
- JP-A- 11 098 978
- JP-A- 2000 239 175
- JP-A- 2001 163 799
- JP-A- 2003 081 868
- JP-A- 2003 135 026
- US-A- 4 524 136
- US-A- 5 656 268
- DATABASE WPI Week 200281 Thomson Scientific, London, GB; AN 2002-744798 XP002509710 & JP 2002 241289 A (ICHIMARU PHARCOS INC.) 28 August 2002 (2002-08-28)
- DATABASE WPI Week 200403 Thomson Scientific, London, GB; AN 2004-025952 XP002509711 & JP 2003 146886 A (NOEVIR KK) 21 May 2003 (2003-05-21)
- DATABASE WPI Week 200348 Thomson Scientific, London, GB; AN 2003-508265 XP002509712 & JP 2002 363054 A (MARUZEN SEIYAKU KK) 18 December 2002 (2002-12-18)

## Description

The present invention relates to methods for moisturizing skin, promoting filaggrin synthesis and remedying dry skin according to claims 1-3.

Manifestation of a moisturizing effect to promote moisture retention in skin tissues are known to include moisture retention in the corneal layer of epidermis by the increase of what is called natural moisturizing factors, which are mainly composed of particular amino acids; moisture retention in the corneocytes by the increase of intercellular lipids, which act as a barrier for retaining moisture in the corneal layer; prevention of washout of the natural moisturizing factors; and the like. The amino acids constituting the essential part of the natural moisturizing factors are supplied by digestion of filaggrin, which is a protein produced by epidermal keratinocytes. Thus promotion of filaggrin production plays an important role in the manifestation of the moisturizing effect. On the other hand, intercellular lipids, which include a variety of lipids, are generated by a number of enzymatic reactions in the epidermis. Among others, ceramide generation is particularly important, wherein formation of a sphingosine skeleton by serine palmitoyl transferase plays an important role.

Many researches have hitherto been made for components having a skinmoisturizing effect, particularly in the field of cosmetics and skin preparations for external use. For example, there have been many proposals to add fermented milk whey or purified products thereof to cosmetics or the like, with an expectation of producing a moisturizing effect. Specifically, Patent Publications 1 and 2 teach that external use of fermented milk whey produces a moisturizing effect. Patent Publication 3 discloses that a skin preparation for external use containing proteinase and a culture supernatant of lactic acid bacteria has a moisturizing effect, and *Lactobacillus helveticus* is disclosed as an example of the lactic acid bacteria.

However, no disclosure is found in any references, including this Patent Publication 3, that a moisturizing effect is confirmed in actual external use of *Lactobacillus helveticus* fermented milk whey. Patent Publication 4 discloses that a culture or a metabolite of *Lactobacillus helveticus* MIKI-020 strain (FERM P-13678) has a melanogenesis inhibitory effect and a collagenogenesis promotive effect, but this reference is silent about a moisturizing effect.

Patent Publications 2 and 3 further contain descriptions that a skin preparation for external use containing fermented milk whey may be used for food and beverage. However, there is no indication as to how the skin preparation for external use could be used for food and beverage and what effect is produced by such use.

Apart from fermented milk whey, there have been some proposals of components that produce a moisturizing effect both by oral intake and by external use. However, it is not accepted as a common rule that a component confirmed to have an effect in one route should exhibit the similar effect also in the other route. There are many components that do not have comparable effects in both of the routes.
Patent Publication 1: JP-2001-31557-A
Patent Publication 2: JP-11-228339-A
Patent Publication 3: WO-01-019324-A
Patent Publication 4: JP-2002-80340-A

Topical cosmetic compositions for moisturizing skin including milk fermented with Lactobacillus spp. are disclosed in US 4,524,136.

Compositions promoting the synthesis of filaggrin are disclosed in WO 99/47117.

According to the present invention, there is provided a method for moisturizing skin comprising orally administering fermented milk whey obtained by fermentation of milk with *Lactobacillus helveticus,* to a subject.

The active component of the skin moisturizer for oral intake and the functional food and beverage containing the moisturizer according to the present invention, is fermented milk whey obtained by fermentation of milk with *Lactobacillus helveticus,* of which safety has been confirmed through oral intake. Thus, an excellent moisturizing effect may be achieved by the oral route with safety, and continuous intake may be expected to provide suppression of skin dryness caused by change of the seasons or climate, and improvement in skin conditions.

The present invention will now be explained in detail.

The skin moisturizer for oral intake according to the methods of the present invention contains, as an active component, fermented milk whey obtained by fermentation of milk with *Lactobacillus helveticus.*

It is preferred to use a strain of *Lactobacillus helveticus* having high extracellular proteinase activity. For example, strains having a U/OD590 value of not lower than 400 are preferred, as measured in accordance with the method of Yamamoto et al. (Yamamoto N. et al., J. Biochem. (1993) 114, 740) based on the method of Twining et al. (Twining, S., Anal. Biochem. 143 3410 (1984)).
Specifically, strains of *Lactobacillus helveticus* having the following bacteriological properties may be used.

### Bacteriological Properties

1. Morphological Properties
   1) Shape of cell: rod
   2) Motility: none
   3) Spore formation: none
   4) Gram stain: positive
2. Physiological Properties
   1) Catalase production: negative
   2) Indole production: negative
   3) Nitrate reduction: negative
   4) Aerobic growth: facultative anaerobic
   5) Formation of DL(-)lactic acid from glucose by homolactic fermentation without formation of gases
   6) Carbohydrate degradation:
      glucose: +
      lactose: +
      mannose: +
      fructose: +
      galactose: +
      sucrose: -
      maltose: -
      xylose: -
      rhamnose: -
      cellobiose: -
      trehalose: -
      melibiose: -
      raffinose: -
      stachyose: -
      mannitol: -
      sorbitol: -
      esculin: -
      salicin: -

An example of such preferred strains of *Lactobacillus helveticus* is *Lactobacillus helveticus* CM-4 (deposited at International Patent Organism Depositary, National Institute of Advanced Industrial Science and Technology, Tsukuba Central 6, 1-1-1 Higashi, Tsukuba-shi, Ibaraki, Japan, under accession number FERM BP-6060 on August 15, 1997) (referred to as CM-4 hereinbelow). CM-4 has been deposited under the above-mentioned accession number under the Budapest Treaty on the International Recognition of the Deposit of Microorganisms for the Purposes of Patent Procedure, and has already been patented.

The fermented milk whey may be obtained by adding a fermented milk starter containing *Lactobacillus helveticus* to milk, suitably selecting fermentation conditions, such as temperature, and fermenting the milk under the selected conditions.

The fermented milk whey as an active component, may be obtained by separating whey from the resulting fermented milk through an ordinary separation process, such as centrifugation or filtration. The fermented milk per se without separation, or separated whey may be used with or without suitable fractionation, concentration, purification, or the like, or the whey or its concentrate may be powdered by lyophilization or spray drying.

The *Lactobacillus helveticus* is preferably in the form of a pre-cultured starter having sufficiently high activity. The initial cell count may preferably be about 10⁵-10⁹ cells/ml.

The fermented milk whey as an active component may also be obtained by cofermentation with *Lactobacillus helveticus* and a yeast, for improved flavor and palatability, when the fermented milk whey is to be used in functional food and beverage, such as foods for specified health uses. The strain of the yeast is not particularly limited, and may preferably be, for example, yeast of the genus *Saccharomyces,* such as *Saccharomyces cerevisiae.* The content of the yeast may suitably be selected depending on the purpose.

The starting material milk may be, for example, animal milk, such as cow's milk, horse's milk, sheep's milk, or goat's milk; vegetable milk, such as soybean milk; or processed milk thereof, such as skim milk, reconstituted milk, powderedmilk, orcondensedmilk. Among these, cow's milk, soybeanmilk, and processed milk thereof are preferred, with cow's milk and its processed milk being particularly preferred.

The solid content of the milk is not particularly limited, and, for example, for skim milk, the solid-non-fat content may usually be about 3 to 15 wt%, and may be 6 to 15 wt% in the light of productivity.

The fermentation may be performed usually by static or stirred culture, for example at 25 to 45 °C, preferably 30 to 45 °C, for 3 to 72 hours, preferably 12 to 36 hours, and the fermentation may be terminated when the lactic acid acidity reaches 1.5 or higher.

The moisturizer according to the methods of the present invention may optionally contain, in addition to the active component fermented milk whey, other conventional components having a moisturizing effect, and various additives, such as excipients, depending on its form.

The essential active component of the moisturizer according to the methods of the present invention is the fermented milk whey, and its oral dosage may suitably be selected so that a desired effect may be achieved, taking into consideration of the duration of intake, continuity, or the like factors. The daily dose is usually 1 to 1000 ml of the fermented milk whey per person, preferably 10 to 200 ml per person.

The moisturizer according to the methods of the present invention may be taken even after the symptoms, such as decrease in skin moisture, are developed, or in the seasons to prevent such symptoms, either continuously daily or intermittently.

The functional food and beverage according to the methods of the present invention are food and beverage containing the present moisturizer, and may be provided, for example, as food for specific health uses that claims prevention or improvement with regard to moisture retention.

The present functional food and beverage may optionally contain additives, such as sugars, proteins, lipids, vitamins, minerals, flavoring agents, or mixtures thereof. Further, the milk components from which the fermented milk whey is separated, may also be contained.

In the functional food and beverage according to the methods of the present invention, the content of the present moisturizer may suitably be selected depending on the form or kind of the food and beverage. The content may suitably be selected also depending on the continuity of intake of the functional food and beverage or the like factors, and is not particularly limited. A suitable content may be usually 0.1 to 100 wt%, preferably 10 to 90 wt% in terms of the active component fermented milk whey.

The functional food and beverage may be in the form of, for example, fermented milk products, such as yogurt or lactic acid bacteria beverage, various processed food and beverage containing the fermented milk whey or a concentrate thereof, dry powders, tablets, capsules, granules, or the like.

The dose and the timing of intake of the functional food and beverage of the present invention are not particularly limited, and it is preferred to take the functional food and beverage in such an amount that the above-mentioned dose of the active component is generally achieved. For example, the present functional food and beverage may be taken continuously or intermittently before or after exposure to the environment that calls for skin moisturization.

The present invention will now be explained in more detail with reference to the examples, which are illustrative only and do not intend to limit the present invention.

### Example 1 and Comparative Examples 1 to 2

### (Preparation of Fermented Milk Whey and Lactic Acid Aqueous Solution)

A milk medium composed of skim milk with a 9 wt% solid content was inoculated with 3% of CM-4 starter (cell count: 5 × 10⁸ cells/mL), and fermented under static conditions at 37 °C for 24 hours to obtain a fermented milk. The fermented milk was centrifuged at 12000 G for 20 minutes, and the precipitate was removed to thereby obtain a fermented milk whey (referred to as fermented milk whey (A) hereinbelow).

For comparison, a 2.67 w/w% lactic acid aqueous solution (referred to as lactic acid aqueous solution (a) hereinbelow) was prepared (Comparative Example 1).

### (Evaluation of Ability to Promote Synthesis of SPT and Filaggrin)

(1) Epidermic cells (normal human keratinocytes, manufactured by KURABO INDUSTRIES LTD.) were cultured in HuMedia-KG2 (trade name, manufactured by KURABO INDUSTRIES LTD.), and subjected to the tests. A 35 mm culture dish was seeded with the epidermic cells at a cell concentration of 5 × 10⁵ cells per dish, and subjected to culture for 24 hours. Then the medium was replaced with a same medium containing fermented milk whey (A) or lactic acid aqueous solution (a) at a concentration shown in Table 1, and further fermented for 24 hours.
(2) After the culture, the cells were washed with PBS (-), and lysed with 500 µL per dish of TRIzol reagent (trade name, manufactured by Gibco BRL). 100 µL per dish of chloroform was added and mixed thoroughly. The supernatant aqueous phase was recovered by centrifugation, and RNAs were precipitated with 2-propanol to obtain the total RNAs. The obtained total RNAs were washed with 75% cold ethanol, and dissolved in diethyl pyrocarbonate (DEPC)-treated water.
(3) 1 µg of the RNAs dissolved in DEPC-treated water was subjected to RT-PCR using One step RT-PCR kit (trade name, manufactured by Qiagen) in accordance with its protocol under the conditions and with the primers shown in Table 2, to reverse transcript and amplify the cDNA corresponding to serine palmitoyl transferase (SPT) mRNA, filaggrin mRNA, or cyclophilin RNA.
(4) After the reaction, the reaction product was fractionated by 1% agarose gel electrophoresis. The electrophoresis gel was stained with ethidium bromide, and photographed under UV irradiation on a transilluminator, to determine the band intensity.
(5) The intensity of the band derived from SPT mRNA or from filaggrin mRNA was standardized by dividing the same by the intensity of the band derived from cyclophilin mRNA, which is believed to be generated in the same amount in any cells, and the obtained value was expressed in a relative value with respect to the value obtained for 0% concentration of the fermented milk whey in the medium in the above paragraph (1) (control), which was taken as 100. The test was conducted twice. The results of each test are shown in Table 1.
(6) Separately, the epidermic cells were cultured in a HuMedia-KG2 medium containing fermented milk whey (A) or lactic acid aqueous solution (a) at various concentrations achieved by serial dilution, and the maximum concentration in the medium of fermented milk whey (A) or lactic acid aqueous solution (a) that did not exhibit cytotoxicity was determined. The maximum concentration was 1.25 % for both cases.

**Table 1**

| | Concentration (v/v%) | Effect on expression of SPT mRNA of normal human epidermic cells | | Effect on expression of filaggrin mRNA of normal epidermic cells | |
|---|---|---|---|---|---|
| | | 1st Test | 2nd Test | 1st Test | 2nd Test |
| Control | 0 | 100 | 100 | 100 | 100 |
| Comparative Example 1 Lactic acid aqueous solution (a) | 0.63 | 91 | 111 | 15 | 132 |
| | 1.25 | 84 | 129 | 87 | 116 |
| Example 1 Fermented milk whey (A) | 0.63 | 153 | 171 | 509 | 196 |
| | 1.25 | 156 | 154 | 543 | 168 |

**Table 2**

| Gene | Sequence | | PCR Conditions | |
|---|---|---|---|---|
| | | | Temperature (°C) | Cycle |
| SPT | Sense | 5'-GAG GCT CAC AGC ATT GGC GC-3' | 58 | 30 |
| | Antisense | 5'-GGC CTG TCC AGT AGA GGT AC-3' | | |
| Filaggrin | Sense | 5'-CAA GCA GAG AAA CAC GTA ATG AGG-3' | 56 | 30 |
| | Antisense | 5'-CGC ACT TGC TTT ACA GAT ATC AGA-3' | | |
| Cyclophilin | Sense | 5'-CCG GGT GAT CTT TGG-3' | 58 | 30 |
| | Antisense | 5'-GGC GAT GGC AAA GGG-3' | | |

### (Preparation of Beverage Containing Fermented Milk Whey (A) and Beverage Containing Non-fermented Milk Whey)

For making fermented milk whey (A) prepared above suitable for drinking, 90 parts by weight of fermented milk whey (A), 0.25 parts by weight of flavoring agents, 0.05 parts by weight of aspartame, and 9.70 parts by weight of water were mixed to prepare beverage containing fermented milk whey (A) (Example 1).

For comparison, to skim milk with a 9 wt% solid content used above for preparation of fermented milk whey (A), lactic acid was added to the acidity of 2.2 %. The mixture was centrifuged at 12000 G for 20 minutes to remove the solid, thereby preparing non-fermented milk whey. Then 90 parts by weight of the obtained non-fermented milk whey, 0.25 parts by weight of flavoring agents, 0.05 parts by weight of aspartame, and 9.70 parts by weight of water were mixed to prepare beverage containing non-fermented milk whey (Comparative Example 2).

### (Evaluation in Oral Intake)

Thirty-two male panels at 24 to 43 years of age with the average age of 29.4 years old, were measured for the epidermal moisture content on the right cheek. According to the results, the panels were divided into two groups of 16 panels each so that the two groups have approximately the same average of the measured values.

One of the groups of panels were given the beverage containing fermented milk whey (A), and the other were given the beverage containing non-fermented milk whey, both in the amount of 150 g per day per person continuously for three weeks, and the following evaluations were made.

### (Evaluation of Epidermal Moisture Content)

The panels were measured for the epidermal moisture content on the right cheek using SKICON 200 (trade name, manufactured by I.B.S. Co., Ltd.) before and three weeks after the intake of the respective beverage. The measurements were made three times, and the average of the results was taken as the epidermal moisture content of that region. The results are shown in Table 3.

The panels of each group were divided into those of dry skin and of normal skin, and the results before and three weeks after the intake were shown separately in Table 4.

**Table 3**

| | Before intake | Three weeks after intake |
|---|---|---|
| | Mean ± SD | Mean ± SD |
| Beverage containing fermented milk whey (A) (Example 1) | 128.8 ± 56.4 | 90.3 ± 76.7 |
| Beverage containing non-fermented milk whey (Comparative Example 2) | 117.5 ± 87.7 | 53.6 ± 35.5 |

| | | |
|---|---|---|
| unit: µS | | |

**Table 4**

| | | Before intake | Three weeks after intake |
|---|---|---|---|
| | | Mean ± SD | Mean ± SD |
| Beverage containing fermented milk whey (A) (Example 1) | Dry skin | 82.8 ± 30.2 | 85.3 ± 86.6 |
| | Normal skin | 169.0 ± 40.1 | 94.8 ± 72.7 |
| Beverage containing non-fermented milk whey (Comparative Example 2) | Dry skin | 69.4 ± 41.9 | 48.0 ± 28.1 |
| | Normal skin | 197.7 ± 87.0 | 62.9 ± 46.8 |

| | | | |
|---|---|---|---|
| unit: µS | | | |

From Table 3, it is seen that the skin moisture content was lowered with the lapse of time compared to that before the oral intake both in Example 1 and Comparative Example 2, which is estimated to be due to the change of the seasons. In the light of this, it is seen that the reduction rate in skin moisture content is lower in Example 1 than in Comparative Example 2. It is thus demonstrated in this test that intake of the beverage containing fermented milk whey (A) inhibits lowering of the epidermal moisture content, i.e., gives a skin moisturizing effect.

From Table 4, it is demonstrated that a similar skin moisturizing effect is provided for both those having dry skin and those having normal skin.

### Referential Example

### (Preparation of Cream)

3.00 parts by weight of 1, 3-butylene glycol, 0.10 parts by weight of p-hydroxybenzoate ester (methylparaben), 40.00 of carboxyvinyl polymer (trade name Carbopol 980, manufactured by NIKKO CHEMICALS CO., LTD.), 5.00 parts by weight of fermented milk whey (A), and 25.90 parts by weight of water were stirred to mix, 12.00 parts by weight of a 10 wt% L-arginine aqueous solution and 14.00 parts by weight of water were added, and homogenously mixed to prepare a cream containing fermented milk whey (A) (referred to as whey(A)-containing cream hereinbelow).

On the other hand, 3.00 parts by weight of 1, 3-butylene glycol, 0.10 parts by weight of p-hydroxybenzoate ester (methylparaben), 40.00 parts by weight of carboxyvinyl polymer (trade name Carbopol 980, manufactured by NIKKO CHEMICALS CO., LTD.), and 30.90 parts by weight of water were stirred to mix, 12.00 parts by weight of a 10 wt% L-arginine aqueous solution and 20.00 parts by weight of water were added, and homogeneously mixed to prepare a cream without fermented milk whey (referred to as comparative cream hereinbelow).

### (Evaluation through Application)

Fifteen male panels at 24 to 43 years of age with the average age of 31.4 years old, were measured for the epidermal moisture content on the right cheek. According to the results, the panels were divided into two groups of 8 or 7 panels each so that the two groups have approximately the same average of the measured values.

One of the groups of panels were had to apply the whey(A)-containing cream, and the other were had to apply the comparative cream, both on the right cheek as will be discussed later, twice a day, in the morning and in the afternoon, continuously for three weeks, and the following evaluation was made.

### (Evaluation of Epidermal Moisture Content)

The panels were measured for the epidermal moisture content on the right cheek using SKICON 200 (trade name, manufactured by I.B.S. Co., Ltd.) before and three weeks after the application of the respective cream. The measurements were made three times, and the average of the results was taken as the epidermal moisture content of that region. The results are shown in Table 5.

**Table 5**

| | Before application | Three weeks after application |
|---|---|---|
| | Mean ± SD | Mean ± SD |
| Whey(A)-containing cream | 142.1 ± 100.3 | 72.4 ± 59.5 |
| Comparative cream | 144.9 ± 97.6 | 77.8 ± 46.9 |

From Table 5, it is seen that the inhibitory effect on lowering in epidermal moisture content was not observed for the whey(A)-containing cream as for the comparative cream without fermented milk whey (A). It is thus demonstrated that fermented milk whey (A) does not produce a moisturizing effect through application on the skin.

## Claims

1. A method for moisturizing skin comprising orally administering fermented milk whey obtained by fermentation of milk with *Lactobacillus helveticus,* to a subject, provided that the method is non-therapeutic.

2. A method for promoting filaggrin synthesis comprising orally administering fermented milk whey obtained by fermentation of milk with *Lactobacillus helveticus,* to a subject, provided that the method is non-therapeutic.

3. A method for remedying dry skin comprising orally administering fermented milk whey obtained by fermentation of milk with *Lactobacillus helveticus,* to a subject, provided that the method is non-therapeutic.

4. The method according to claim 1, wherein said *Lactobacillus helveticus* is *Lactobacillus helveticus* CM-4 (deposited at International Patent Organism Depositary, National Institute of Advanced Industrial Science and Technology under accession number FERM BP-6060).

5. The method according to claim 2, wherein said *Lactobacillus helveticus* is *Lactobacillus helveticus* CM-4 (deposited at International Patent Organism Depositary, National Institute of Advanced Industrial Science and Technology under accession number FERM BP-6060).

6. The method according to claim 3, wherein said *Lactobacillus helveticus* is *Lactobacillus helveticus* CM-4 (deposited at International Patent Organism Depositary, National Institute of Advanced Industrial Science and Technology under accession number FERM BP-6060).

## Patentansprüche

1. Verfahren zur Feuchtigkeitsversorgung der Haut, das die orale Verabreichung fermentierten Milchserums, das durch Fermentierung von Milch mit *Lactobacillus helveticus* erhalten wird, an ein Individuum umfasst, mit der Maßgabe, dass das Verfahren nicht therapeutisch ist.

2. Verfahren zur Förderung der Filaggrin-Synthese, das die orale Verabreichung fermentierten Milchserums, das durch Fermentierung von Milch mit *Lactobacillus helveticus* erhalten wird, an ein Individuum umfasst, mit der Maßgabe, dass das Verfahren nicht therapeutisch ist.

3. Verfahren zur Abhilfe bei trockener Haut, das die orale Verabreichung fermentierten Milchserums, das durch Fermentierung von Milch mit *Lactobacillus helveticus* erhalten wird, an ein Individuum umfasst, mit der Maßgabe, dass das Verfahren nicht therapeutisch ist.

4. Verfahren nach Anspruch 1, wobei der *Lactobacillus helveticus* der *Lactobacillus helveticus* CM-4 (hinterlegt bei der International Patent Organism Depositary, National Institute of Advanced Industrial Science and Technology unter der Hinterlegungsnummer FERM BP-6060) ist.

5. Verfahren nach Anspruch 2, wobei der *Lactobacillus helveticus* der *Lactobacillus helveticus* CM-4 (hinterlegt bei der International Patent Organism Depositary, National Institute of Advanced Industrial Science and Technology unter der Hinterlegungsnummer FERM BP-6060) ist.

6. Verfahren nach Anspruch 3, wobei der *Lactobacillus helveticus* der *Lactobacillus helveticus* CM-4 (hinterlegt bei der International Patent Organism Depositary, National Institute of Advanced Industrial Science and Technology unter der Hinterlegungsnummer FERM BP-6060) ist.

## Revendications

1. Procédé d'hydratation de la peau comprenant l'administration orale de petit-lait fermenté obtenu par fermentation de lait avec *Lactobacillus helveticus* à un sujet, dans la mesure où le procédé est non thérapeutique.

2. Procédé de stimulation de la synthèse de filaggrine comprenant l'administration orale de petit-lait fermenté obtenu par fermentation de lait avec *Lactobacillus helveticus* à un sujet, dans la mesure où le procédé est non thérapeutique.

3. Procédé de lutte contre la peau sèche comprenant l'administration orale de petit-lait fermenté obtenu par fermentation de lait avec *Lactobacillus helveticus* à un sujet, dans la mesure où le procédé est non thérapeutique,

4. Procédé selon la revendication 1, où ledit *Lactobacillus helveticus* est *Lactobacillus helveticus* CM-4 (déposé auprès du International Patent Organism Depositary, National Institute of Advanced Industrial Science and Technology sous le numéro d'accès FERM BP-6060).

5. Procédé selon la revendication 2, où ledit *Lactobacillus helveticus* est *Lactobacillus helveticus* CM-4 (déposé auprès du International Patent Organism Depositary, National Institute of Advanced Industrial Science and Technology sous le numéro d'accès FERM BP-6060).

6. Procédé selon la revendication 3, où ledit *Lactobacillus helveticus* est *Lactobacillus helveticus* CM-4 (déposé auprès du International Patent Organism Deposltary, National Institute of Advanced Industrial Science and Technology sous le numéro d'accès FERM BP-6060).
